# EUROPÄISCHE PATENTSCHRIFT

(11) **EP 1 873 236 B1**
(45) Veröffentlichungstag und Bekanntmachung des Hinweises auf die Patenterteilung: **07.11.2012**
(21) Anmeldenummer: 07011691.8
(22) Anmeldetag: 14.06.2007
(51) Int. Cl.: C12N 5/077, G01N 33/50, G01N 33/68

(54) **Verfahren zur Qualitätskontrolle von in einem Kulturmedium kultivierten Zellen mittels SERPINA1als Qualitätsmarker für die chondrogene Potenz von Chondrozyten**
Quality control method for cells cultivated in a culture medium with SERPINA1 as quality markers for chondrogenic potency of chondrocytes
Procédé destiné au contrôle de la qualité de cellules cultivées dans un milieu de culture et SERPINA1 en tant que marqueurs qualitatifs pour la puissance condrogène des condrozytes

(30) Priorität: 14.06.2006 DE 102006027991
(43) Veröffentlichungstag der Anmeldung: 02.01.2008
(73) Patentinhaber: Universitätsklinikum Heidelberg, 69120 Heidelberg (DE)
(72) Erfinder: Richter, Wiltrud, Prof. Dr., 69151 Neckargemünd (DE); Pelttari, Karoliina, 69115 Heidelberg (DE); Boeuf, Stephane, Dr., 69120 Heidelberg (DE)
(74) Vertreter: Ullrich & Naumann

(56) Entgegenhaltungen:
- WO-A-02/095399
- US-A1- 2003 235 813
- FISCHER DAGMAR-CHRISTIANE ET AL: "Induction of alpha1-antitrypsin synthesis in human articular chondrocytes by interleukin-6-type cytokines: Evidence for a local acute-phase response in the joint" ARTHRITIS AND RHEUMATISM, Bd. 42, Nr. 9, September 1999 (1999-09), Seiten 1936-1945, XP002458120 ISSN: 0004-3591

## Beschreibung

Die Erfindung betrifft ein Verfahren zur Qualitätskontrolle von in einem Kulturmedium kultivierten Zellen, wobei es sich bei den kultivierten Zellen um *in vitro* kultivierte Chondrozyten handelt. Des Weiteren betrifft die Erfindung die Verwendung von SERPINA 1 als Qualitätsmarker.

Zur Regeneration von Gewebeschäden wird in Kliniken neben den konventionellen Heil- und Behandlungsverfahren auch das Verfahren der autologen Zell-Transplantation eingesetzt. Dazu werden körpereigene Zellen des zu behandelnden Patienten entnommen und diese im Labor angezüchtet.

So ist zum Beispiel die autologe Chondrozyten Transplantation (ACT) ein in der Klinik eingesetztes Verfahren zur biologischen Rekonstruktion von fokalen Knorpelschäden, wobei entnommene körpereigene Chondrozyten *in vitro* expandiert werden, um adäquate Zellzahlen zu erreichen.

Als ein besonderes Problem stellt sich bei der künstlichen Anzüchtung der Zellen deren Eigenschaften zur Dedifferenzierung dar. Das bedeutet, dass die Zellen im Laufe der Anzüchtung ihre gewebsspezifischen Eigenschaften verlieren und möglicherweise bei der Transplantation in dem zu behandelnden Gewebe nicht die gewünschten Eigenschaften aufweisen. Dies ist insoweit von Nachteil, als dadurch der Behandlungserfolg in Frage gestellt werden muss.

Es ist beispielsweise bekannt, dass Chondrozyten in Abhängigkeit der Kultivierungsweise, also in Abhängigkeit von Faktoren wie z.B. der Kultivierungsdauer, der Zusammensetzung des Kulturmediums, hier insbesondere in Abhängigkeit vom Serumgehalt und der Zugabe von Wachstumsfaktoren, während der Anzüchtung ihre knorpelspezifischen Eigenschaften verlieren. Dies verhindert ein hochwertiges Rekonstruktionsergebnis nach der Transplantation der expandierten Zellen im geschädigten Knorpelgewebe des Patienten.

Ein hochwertiges Rekonstruktionsergebnis kann also nur gewährleistet werden, wenn die bei der Transplantation implantierten Zellen weiterhin ihre zellspezifischen Eigenschaften aufweisen. So kann beispielsweise ein hochwertiges Rekonstruktionsergebnis in einem geschädigten Knorpelgewebe nur dann gewährleistet werden, wenn die implantierten Knorpelzellen bei ihrer Kultivierung die Fähigkeit zur Synthese knorpelrelevanter Matrixkomponenten behalten, damit sie sich in das geschädigte Knorpelgewebe einfügen können.

Es ist daher zwingend notwendig vor der Transplantation der Zellen die Vitalität der Zellen sowie deren Qualität in Hinsicht auf ihre zellspezifischen Eigenschaften in der *in vitro*-Kultur zu überprüfen.

Für die Überprüfung der Qualität von Chondrozyten wurde ein Verfahren vorgeschlagen, bei dem gezielt die Analyse der Gene Kollagen Typ II, Fibroblasten-Wachstumsfaktor-Rezeptor 3 (FGFR3), Aggrekan und knorpelrelevante Wachstumsfaktoren, wie z.B. BMP-2 (Bone Morphogenetic Protein-2) auf Basis der mRNS der Zellen durchgeführt wird [Dell'Accio F et al.. Molecular markers predictive of the capacity of expanded human articular chondrocytes to form stable cartilage in vivo. Arthritis Rheum 2001; 44: 1608-19].

Allerdings müssen für diese Überprüfungen Zellen aus der angezüchteten Kultur "geopfert" werden, um mRNS (messenger Ribonukleinsäure) aus den entnommenen Zellen isolieren zu können.

Nachteilig an dieser Methode ist daher zum einen die Tatsache, dass Zellen aus der Kultur entnommen werden müssen, also ein Verbrauch von Zellen hingenommen werden muss, um mRNS extrahieren zu können. Zum anderen ist die Extraktion von mRNS sehr aufwendig und zeitintensiv. Des Weiteren handelt es sich bei mRNS um ein sehr instabiles Molekül, so dass es bei der Lagerung und der Aufarbeitung zu Verlusten kommen kann, wodurch eine Standardisierung extrem schwer fällt. Daher ergibt sich eine schlechte Korrelationsmöglichkeit zwischen der Quantität der mRNS für die oben beschriebenen Gene zu der chondrogenen Potenz der angezüchteten Chondrozyten.

Aus US 2003/235813 A1 ist die Identifizierung von Markern zur Beurteilung der phänotypischen Stabilität von Chondrozyten bekannt. Als geeigneter Marker ist hier Stromelysin (=MMP3) identifiziert. Die Marker können mittels Antikörpern auf Proteinebene gemessen werden.

Aus WO 02/095399 A ist ein Verfahren zur Qualitätskontrolle für Chondrozyten bekannt, wobei als Marker u.a. Cytokine und Wachstumsfaktoren wie TGF-beta-3 und BMP-2 verwendet werden. Die Marker werden mit Immunofluoreszenz oder Western Blot bestimmt.

Der vorliegenden Erfindung liegt nun die Aufgabe zugrunde, ein Verfahren zur Qualitätskontrolle von in einem Kulturmedium kultivierten Zellen anzugeben wonach ohne Aufarbeitung von Zellmaterial, also ohne Verlust von Zellmaterial eine zuverlässige Aussage über die zellspezifische Potenz der kultivierten Chondrozyten möglich ist. Des Weiteren soll die Verwendung eines besonderen Qualitätsmarkers unter den vorstehenden Voraussetzungen angegeben werden.

Die voranstehende Aufgabe wird durch die Merkmale der nebengeordneten Patentansprüche 1 und 3 gelöst.

Gemäß Patentanspruch 1 ist ein Verfahren zur Qualitätskontrolle von in einem Kulturmedium kultivierten Zellen dadurch gekennzeichnet, dass die Konzentration mindestens eines von den Zellen in das Kulturmedium sekretierten Moleküls zur Messung der chondrogenen Potenz der Zellen mittels einer ELISA-Aufarbeitungim Kulturüberstand gemessen wird, wobei es sich bei dem mindestens einen sekretierten Molekül um den Peptidase-Inhibitor SERPINA1 handelt.

Erfindungsgemäß ist erkannt worden, dass die Messung der Konzentration mindestens eines von den Zellen in das Kultumedium sekretierten Moleküls mit der Qualität der Zellen korreliert werden kann, also Aufschluss über deren zellspezifische Potenz gibt. Zudem ist erkannt worden, dass eine solche Qualitätskontrolle der Zellen auch ohne Verbrauch von Zellmaterial, also ohne Entnahme von Zellen auskommt, nämlich durch die Messung mindestens eines von den Zellen sekretierten Moleküls im Kulturmediumüberstand. Auch ist erkannt worden, dass sich standardisierte, automatisierte Laborverfahren, nämlich die ELISA ("Enzymelinked Immunosorbent Assay")-Technik mit einer Messung im Kulturüberstand, für die Qualitätskontrolle der Zellen einsetzen lassen, so dass sich der Zeitaufwand zum Nachweis der zellspezifischen Potenz gegenüber dem bekannten Verfahren extrem reduzieren lässt.

Es wird ein von den Zellen sekretiertes Eiweißmolekül (Protein) gemessen, wobei mit der zellspezifischen Potenz der Zellen korreliert werden kann. Dies ist dahingehend vorteilhaft, da auf Peptidbibliotheken zurückgegriffen werden kann, die zur Identifikation von Antikörperbindungsstellen ("Epitopen") auf Proteinen dienen. Es ist also mit der ELISA-Technik möglich, spezielle Proteine anhand derer über die Peptidbibliotheken ermittelten Epitope zu messen, die mit der zellspezifischen Potenz der Zellen korreliert werden können.

Um eine Aussage über die Aktivität der Zellen machen zu können, eignet sich besonders der Nachweis von Enzymen, insbesondere von Peptidasen, da diese für alle Organismen lebensnotwendig sind und sie ubiquitär, d. h. in allen Geweben und Zellen vorkommen.

Extrazelluläre, sezernierte Peptidasen findet man zum Beispiel bei tierischen Organismen vor allem im Verdauungstrakt, wo sie die hydrolytische Spaltung von Nahrungsmitteln katalysieren. Sie werden aber auch in anderen extrazellulären Flüssigkeiten gefunden, wo sie zum Teil hochspezifische Aufgaben übernehmen, wie zum Beispiel die Peptidasen des Blutgerinnungssystems, des Komplementsystems und des fibrinolytischen Systems.

So lassen sich im Kulturmedium von Chondrozyten während ihrer *in vitro-*Kultivierung die Konzentrationen von SERPINA1 (Serin- (oder Cystein-) Proteinase-Inhibitor1), auch als alpha 1 Antitrypsin bekannt, messen, die von den Chondrozyten in das Kulturmedium sekretiert werden. Die Konzentration von SERPINA1 gibt dabei Aufschluss über die chondrogene Potenz der Zellen.

Somit kann erstmalig während der *in vitro*-Kultivierung der Chondrozyten zu verschiedenen Zeitpunkten Kulturüberstand abgenommen und die Konzentration von SERPINA1 gemessen werden und gegebenenfalls durch Veränderung der Kulturbedingungen, beispielsweise durch Zugabe von Wachstumsfaktoren in das Kulturmedium oder durch Veränderung des Serumgehaltes des Kulturmediums, auf das Kulturergebnis, nämlich die Qualität der Zellen Einfluss genommen werden. Zudem ist der Zeitpunkt, an dem die Zellen beginnen ihre zellspezifischen Eigenschaften zu verlieren, gegenüber den bekannten Verfahren genauer zu ermitteln, da kurze Intervalle zur Messung eingehalten werden können.

Die Messung der Konzentration von SERPINA1 im Kulturüberstand dient somit hervorragend zur Qualitätssicherung von *in vitro* expandierten Chondrozyten in Hinsicht auf deren chondrogene Potenz, wobei die Entnahme von Zellen zur Aufarbeitung von mRNS, also der Verbrauch von Zellen vermeidbar ist.

Dadurch wird die Qualität der biologischen Rekonstruktion fokaler Knorpelschäden also das Rekonstruktionsergebnis bei der autologen Chondrozyten Transplantation in zweifacher Hinsicht positiv beeinflusst. Zum einen lässt sich über die Messung der Konzentration von SERPINA1 als ein standardisierbares Nachweisverfahren mit hervorragender Korrelation zu den gewebsspezifischen Eigenschaften die chondrogene Potenz der kultivierten Chondrozyten, also die Qualität der Zellen zu jedem Zeitpunkt der Kultivierung kontrollieren und somit der bestmögliche Zeitpunkt für die Transplantation bestimmen. Zum anderen kann für die biologische Rekonstruktion fokaler Knorpelschäden gegenüber den bekannten Verfahren auf eine erhöhte Anzahl von Zellen zurückgegriffen werden, da für die Qualitätssicherung keine Zellen zur Aufarbeitung von mRNS "geopfert" werden müssen.

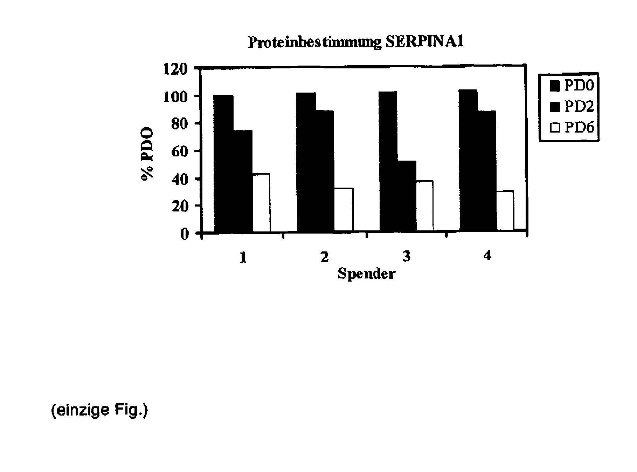

Es gibt nun verschiedene Möglichkeiten, die Lehre der vorliegenden Erfindung in vorteilhafter Weise auszugestalten und weiterzubilden. Dazu ist einerseits auf die nachgeordneten Ansprüche, andererseits auf die nachfolgende Erläuterung bevorzugter Ausführungsbeispiele des erfindungsgemäßen Verfahrens und der erfindungsgemäßen Qualitätsmarker anhand der Zeichnung und des Beispiels zu verweisen. In Verbindung mit der Erläuterung der bevorzugten Ausführungsbeispiele werden auch im Allgemeinen bevorzugte Ausgestaltungen und Weiterbildungen der Lehre erläutert, ohne die Lehre darauf einzuschränken.

In der Zeichnung zeigt die
- einzige Fig.: in einer Grafik das Ergebnis der Proteinbestimmung von SERPINA1 zu verschiedenen Zeitpunkten in den Kulturüberständen von aus 4 Spendern entnommenen und *in vitro* kultivierten Chondrozyten.

Die einzige Fig. zeigt das Ergebnis des erfindungsgemäßen Verfahrens für eine standardisierte Proteinbestimmung des erfindungsgemäßen Qualitätsmarkers SERPINA1 in verschiedenen Chondrozytenkulturen aus 4 Spendern zu 3 unterschiedlichen Zeitpunkten; PD0: mit frisch isolierten Zellen (keine Populationsverdoppelung), PD2: nach 2 Populationsverdoppelungen und PD6: nach 6 Populationsverdoppelungen,

Gemäß der einzigen Fig. sind die Proteinkonzentrationen von SERPINA1 prozentual in Relation zu den Anfangskonzentrationen ausgedrückt. Zellen nach 6 Populationsverdoppelungen (PD6), bei denen der Spiegel von SERPINA1 unter 45% des Anfangsspiegels (PDO) des Anfangsspiegels gemessen wurde, haben ihre Fähigkeit zur ektopen Knorpelbildung verloren. Bei den Zellen mit höheren Spiegeln ist die ektope Knorpelbildung erfolgreich.

(Zur Evaluierung der Knorpelbildungsfähigkeit wurden die Zellen nach der Bestimmung der Konzentration von SERPINA1 in immun-defiziente Mäuse subkutan transplantiert).

Aus dem Ergebnis der Knorpelbildungsfähigkeit in den immun-defizienten Mäusen konnte eine Korrelation zwischen den Spiegeln der Proteine SERPINA1 in den Kulturüberständen und der chondrogenen Potenz der kultivierten Zellen ermittelt werden.

Insofern zeigt die einzige Fig. Werte für die Proteinbestimmung von SERPINA1 an, deren Spiegel mit der chondrogenen Potenz der kultivierten Zellen korreliert werden können.

Das nachfolgende Beispiel gibt die Bestimmung des Anfangswertes und die Bestimmung des Wertes bei der Transplantation, also die Bestimmung der Konzentration von SERPINA1 in Kulturüberständen zu verschiedenen Zeitpunkten wider.

### Beispiel

### a) Bestimmung des Anfangswertes

Durch enzymatischen Verdau werden Chondrozyten aus dem Gelenkknorpel eines Patienten isoliert und deren Zellzahl bestimmt. Für eine standardisierte Qualitätskontrolle wird eine definierte Menge von Zellen in eine Kulturschale definierter Größe über einen definierten Zeitraum mit einem definiertem Volumen Medium inkubiert. Beispielsweise werden 500.000 Zellen in einer 6 Well-Platte mit 4 ml Medium ausgesät und nach 4 Tagen Kultivierungsdauer Kulturüberstand für Analysen entnommen. Mittels ELISA wird dann in dem entnommenen Medium die Konzentration von SERPINA1 (SERPINA1: ELISA Kit der Firma Immundiagnostik) bestimmt.

### b) Bestimmung des Wertes bei der Transplantation

Nach der Isolation werden die Zellen expandiert, bis eine ausreichende Zellzahl für eine Transplantation erreicht wurde. Vier Tage vor dem Erreichen dieser Zellzahl, d.h. vor Ernten für die Transplantation wird dieselbe definierte Zellzahl wie zu Beginn der Kultur ausgesät, um dann am Tag der Transplantation den Kulturüberstand für die Proteinbestimmung bereitstellen zu können. Die Proteinbestimmung erfolgt dann, wie für den Anfangswert beschrieben, unter Anwendung der ELISA-Technik und den beschriebenen Kits.

## Patentansprüche

1. Verfahren zur Qualitätskontrolle von in einem Kulturmedium kultivierten Zellen, wobei es sich bei den kultivierten Zellen um *in vitro* kultivierte Chondrozyten handelt,
**dadurch gekennzeichnet, dass** die Konzentration mindestens eines von den Zellen in das Kulturmedium sekretierten Moleküls zur Messung der chondrogenen Potenz der Zellen mittels einer ELISA-Aufarbeitung im Kulturüberstand gemessen wird, wobei es sich bei dem mindestens einen sekretierten Molekül um den Peptidase-Inhibitor SERPINA1 handelt.

2. Verfahren nach Anspruch 1, **dadurch gekennzeichnet, dass** die Konzentration des mindestens einen Enzyms oder eines Teils dieses Enzyms gemessen wird.

3. Verwendung von SERPINA1 als Qualitätsmarker für die Messung der chondrogenen Potenz von Chondrozyten.

## Claims

1. Method for the quality control of cells cultivated in a culture medium, wherein the cultivated cells are chondrocytes cultivated *in vitro.*
**characterised in that** the concentration of at least one molecule secreted into the culture medium by the cells is measured in the culture supernatant by means of an ELISA workup for measurement of the chondrogenic potency of the cells, wherein the at least one secreted molecule is the peptidase inhibitor SERPINA1.

2. Method according to claim 1, **characterised in that** the concentration of the at least one enzyme or of part of that enzyme is measured.

3. Use of SERPINA1 as quality marker for the measurement of the chondrogenic potency of chondrocytes.

## Revendications

1. Procédé destiné au contrôle de la qualité des cellules cultivées dans un milieu de culture, lesdites cellules cultivées étant des chondrocytes cultivés *in vitro*,
**caractérisé en ce que** la concentration d'au moins une molécule sécrétée par les cellules dans le milieu de culture, destinée à mesurer l'activité chondrogène des cellules, est mesurée au moyen d'un renouvellement de l'ELISA dans la partie surnageante du milieu de culture, ladite au moins une molécule sécrétée étant l'inhibteur de peptidases SERPINA1.

2. Procédé selon la revendication 1, **caractérisé en ce que** l'on mesure la concentration de ladite au moins une enzyme ou d'une partie de cette enzyme.

3. Utilisation du SERPINA1 en tant que marqueur qualitatif pour la mesure de l'activité chondrogène des chondrocytes.
